Europäisches Patentamt

European Patent Office

Office européen des brevets

(19)

(11) Publication number: **0 061 546
B1**

(12)                    EUROPEAN PATENT SPECIFICATION

(45) Date of publication of patent specification: **04.07.84**

(51) Int. Cl.³: **G 01 N 33/54, C 12 Q 1/44**

(21) Application number: **81301377.8**

(22) Date of filing: **30.03.81**

(54) **Reagent for streptococcal anti-esterase assay.**

(43) Date of publication of application:
**06.10.82 Bulletin 82/40**

(45) Publication of the grant of the patent:
**04.07.84 Bulletin 84/27**

(84) Designated Contracting States:
**DE FR GB IT**

(56) References cited:
FR - A - 2 190 917
FR - A - 2 464 301
US - A - 4 169 138

CHEMICAL ABSTRACTS, vol. 82, no. 23, June 9, 1975, page 410, abstract 153769a, COLUMBUS, OHIO (US) & JP - A - 74 132 288 (INSTITUTE OF HYGIENIC SCIENCES, SAITAME PREFECTURE), December 18, 1974

CHEMICAL ABSTRACTS, vol. 71, no. 9, September 1, 1969, page 120, abstract 37139u, COLUMBUS, OHIO (US) A.H. STOCK et al.: "Extracellular esterases of streptococci and the distribution of specific antibodies in human serum of various age groups" & J. Immunol. 1969, 102(4), 859-869

(73) Proprietor: **Dainippon Pharmaceutical Co., Ltd.**
**25, Doshomachi 3-chome Higashi-ku**
**Osaka-shi, Osaka 541 (JP)**

(72) Inventor: **Hayano, Seiki**
**48, Uetake 2-chome**
**Omiya-shi Saitama-ken (JP)**
Inventor: **Yakogawa, Kanae**
**4-17, Aogakidai 3-chome**
**Nara-shi, Nara-ken (JP)**
Inventor: **Kurooka, Shigeru**
**10-7, Nonaka 3-chome**
**Fujiidera-shi Osaka-fu (JP)**

(74) Representative: **Harrison, David Christopher et al,**
**MEWBURN ELLIS & CO 2/3 Cursitor Street**
**London EC4A 1BQ (GB)**

(56) References cited:
HAEMATOLOGY, 1977, 2nd edition, Ed. W.J. Williams et al. NEW YORK (US) D.A. NELSON et al.: "Leukocyte esterases", chapter A25, pages 1633-1636

ARZNEIMITTEL FORSCHUNG, vol. 28, no. 11A, 1978, MANNHEIM (DE) L. SCHROTT: "Enzymimmunoassay", pages 1934-1940

# 0 0 6 1 5 4 6

**Description**

The present invention relates to a reagent for streptococcal anti-esterase assay. More particularly, it relates to a novel reagent useful for the determination of an anti-esterase antibody, i.e. an antibody against an esterase from pathogenic streptococci.

In serodiagnosis of diseases caused by pathogenic streptococcal infections such as rheumatic fever, acute glomerulonephritis and scarlet fever, particularly in case of the diseases caused by infections of Lancefield's hemolytic group A streptococci, it has, hitherto, been done by determining the titer of an antibody against extracellular components. There have usually been determined the antibody against Streptolysin O (ASO) and the antibody against deoxyribonuclease B. Antibody against hyaluronidase, streptokinase or NAD nucleotidase is occasionally determined.

It has been also reported by Hayano to determine an anti-esterase antibody [cf. Seiki Hayano, Infection and Immunity *15* (1), 295—299 (1977)]. According to this method, the anti-esterase antibody and the esterase (antigen) are spotted on an agar gel plate and subjected to a repetitive counter electrophoresis and then the esterase bound to the anti-esterase antibody on the agar gel plate is colored by spraying chromogenic substrate reagent, and the resulting appeared moved spot is measured. The value of anti-esterase antibody is determined based on the size of the colored spot. This method is advantageous in that it can be done even in a small amount of test materials and that the gel plate with colored spots can be kept for a long period of time. However, it has such disadvantages as complicated procedures and time consuming (about 48 hours), and further, the obtained data are not quantitative (rather qualitative) because they are determined from the size of spot.

The present inventors have intensively studied improvement of the determination of an antibody against an esterase from pathogenic streptococci in human blood serum. As a result, it has been found that the antiesterase antibody can quantitatively be determined within a very short period of time such as about one to two hours with a small amount of a test material by the method comprising adding an esterase obtained from a culture broth of a pathogenic streptococcus and a human blood serum into a test tube, adding thereto an insoluble protein being capable of binding to human immunoglobulin, incubating the mixture, separating the resulting "an esterase-anti-esterase antibody-insoluble protein" complex, and then determining the esterase activity thereof with a conventional reagent for determining esterase activity, wherein the esterase activity is in a quantitative relation with the amount of the anti-esterase antibody in the human blood serum.

An object of the present invention is to provide a reagent useful for the improved method for the determination of an antibody against an esterase from pathogenic streptococci, i.e. for streptococcal anti-esterase assay. Another object of the invention is to provide an improved method for the determination of the antiesterase antibody which is useful for serodiagnosis of diseases caused by pathogenic streptococcal infections. These and other objects and advantages of the invention will be apparent to persons skilled in the art from the following description.

The reagent for streptococcal anti-esterase assay of the present invention comprises a kit of the following reagents:

reagent 1: an esterase (a) from pathogenic streptococci,

reagent 2: a protein (b) which is capable of binding to an antibody (d) against the esterase (a) from pathogenic streptococci and is bound to an insoluble carrier, and

reagent 3: a reagent (c) for measuring an activity of the esterase (a).

The esterase (a) (reagent 1, which may occasionally be referred to as "antigen") can be obtained by cultivating a pathogenic streptococcus, fractionating the resulting culture broth containing esterase with ammonium sulfate, subjecting the resulting fraction to dialysis and then to lyophilization. (cf. Japanese Patent Laid Open Application No. 132,288/1974 published on December 18, 1974).

The pathogenic streptococci include hemolytic Group A streptococci (e.g. *Streptococcus pyogenes* 69882, type A 49; and *Streptococcus pyogenes* T4, type 4), hemolytic Group B streptococci (e.g. *Streptococcus* sp. H36B, type B—I—b; and *Streptococcus pyogenes* B III D 136C, type III), or the like. Any other streptococci which are available from various institutes of microorganisms depositories in the world can be used if they have an esterase-producing capacity without regard to having hemolytic properties or not. The esterases (a) are classified into various serological types such as type A—I, type A—II, type B, type C, etc. in accordance with the kinds of pathogenic streptococci, and these various types of esterase can be all used in the present invention. According to the reagent of the present invention, each anti-esterase antibody (d) corresponding to each type of esterase can separately be determined. For example, when the type A—I esterase (a) is used, the anti-type A—I esterase antibody (d) can be determined, but other anti-esterase antibodies such as anti-type A—II esterase antibody and anti-type B esterase antibody can not be determined. In order to determine the anti-type A—II esterase antibody or anti-type B esterase antibody, type A—II esterase or type B esterase should be used, respectively. This differential determination of each anti-esterase antibody is one of the characteristics of the present invention. It is also possible to determine total value of two or more kinds of anti-esterase antibodies by using a mixture of two or more types of esterase as the reagent 1.

The protein (b) which is capable of binding to an anti-esterase antibody (d) and is bound to an

2

insoluble carrier, (reagent 2, which may occasionally be referred to as "immuno-adsorbent") includes, for example, an antibody against an anti-esterase antibody (d) which is bound to a conventional insoluble carrier such as bacterial cell walls, glass beads or Sephadexes. The antibody against an anti-esterase antibody (d) may be obtained by administering parenterally the anti-esterase antibody (d) to an animal different from the animal which produced the antibody (d), but preferably be obtained by administering parenterally an immunoglobulin of an animal which produced the antibody (d) to another animal and separating the produced antibody. The antibody against anti-esterase antibody (d) thus obtained is bound to a conventional insoluble carrier (e.g. bacterial cell walls) with a divalent binding agent (e.g. glutaraldehyde). (cf. Japanese Patent Laid Open Application No. 117,419/1977, published on October 1, 1977).

As a reagent 2, there can be used cell walls of bacteria such as *Staphylococcus aureus* which contain "protein-A". The "protein-A" is a protein which can non-specifically bind to immunoglobulin [cf. Arne Forsgren et al, The Journal of Immunology *97*, 822—827 (1966)]. In the bacterial cell walls containing "protein-A", the moiety of "protein-A" corresponds to the protein being capable of binding to the anti-esterase antibody (d) and the remaining moiety corresponds to the insoluble carrier.

The bacterial cell walls containing "protein-A" can be used as they stand, but alternatively, the "protein-A" is isolated and purified and then is bound to an insoluble carrier such as glass beads or Sephadexes. A "protein-A" bound to a Sephadex is commercially available in the name of "Protein-A" — Sepharose CL—4B" (manufactured and sold by Pharmacia, Sweden).

The reagent (c) for determining an activity of the esterase (reagent 3) includes all conventional reagents which comprises a substrate for the esterase (a) and a coloring agent which can react with hydrolyzed products of the substrate to give a color, for example a combination of S-acetylthiophenol and 5,5'-dithio-bis(2-nitrobenzoic acid) (hereinafter, referred to as "DTNB"), a combination of naphthyl acetate and a diazo-compound (e.g. naphthylamine diazonium chloride), nitrophenyl acetate (this compound functions both as a substrate and as a coloring agent), or the like. The reagent 3 may additionally contain other agents such as an enzymatic stopping reagent [e.g. acetone or acidic Tris-malate buffer (pH 5—6)], a buffering agent (e.g. Tris-HCl buffer or phosphate buffer), or the like.

These reagents 1, 2 and 3 are usually in the form of a kit in the liquid or powder state in accordance with the properties of the reagents.

The determination of an anti-esterase antibody (d) in test material by using the reagent of the present invention can be carried out in the following steps.

*First step* (antigen-antibody reaction)
The reagent 1, i.e. an esterase (a) (antigen), is added to the test material (antibody) and is subjected to an antigen-antibody reaction to give an antigen-antibody complex (e).

*Second step* (Collection of the antigen-antibody complex)
The antigen-antibody complex (e) is reacted with the reagent 2 (immuno-adsorbent) and the resulting precipitate of the complex (e) is collected.

*Third step* (Measurement of esterase activity)
The activity of esterase contained in the precipitate obtained in the second step is measured by using the reagent 3.

The first step can usually be carried out by mixing the test material with the esterase (a) (preferably in an excess amount) in a buffer solution (pH 6—8) such as phosphate buffer and incubating the mixture at a temperature of from 20 to 40°C, preferably about 37°C, for 5 to 40 minutes. The resulting mixture containing an antigen-antibody complex (e) is used in the second step.

The second step can usually be carried out by reacting the mixture containing an antigen-antibody complex (e) obtained in the first step with the reagent 2 (immuno-adsorbent) in a buffer solution (pH 6—8) and incubating the mixture at a temperature of from 20 to 40°C, preferably about 37°C, for 5 to 40 minutes, followed by collecting the resulting precipitate by centrifugation or any other conventional separation means.

The third step can usually be carried out by reacting the precipitate obtained in the second step with a substrate for the esterase (a) and then reacting the resultant with a coloring agent, followed by measuring the resulting colored solution in a conventional manner. For example, the precipitate is reacted with S-acetylthiophenol (substrate) and the resulting thiophenol is reacted with DTNB (which is a thiol group-coloring agent), and the resulting yellow color intensity is measured with a colorimeter.

Alternatively, the determination of the anti-esterase antibody (d) can be carried out by exchanging the first step and the second step. That is, the reagent 2 (immuno-adsorbent) is firstly added to the test material in a buffer solution (pH 6—8) and the mixture is reacted likewise, the resulting precipitate collected by a centrifuge is subjected to the antigen-antibody reaction with the reagent 1 and washed by a centrifuge, followed by the measurement of the activity of esterase (the third step).

Further alternatively, the first and second steps can be done simultaneously. That is, the reagent 1 and the reagent 2 are simultaneously added to the test material in a buffer solution (pH 6—8) and the mixture is subjected to the reaction likewise, and the resulting precipitate of antigen-antibody complex

3

is collected and subjected to the measurement of the activity of esterase (the third step). This alternative method is the most convenient because of the simplest procedure. The results of these three methods are well correlated.

The reagent of the present invention is useful for diagnosis of diseases caused by various pathogenic streptococcal infections, particularly hemolytic streptococcal infections.

The present invention is illustrated by the following Examples but is not limited thereto. In the Examples, "%" is % by weight, unless specified otherwise.

## Example 1

Preparation of esterase (a) (antigen):

A culture medium (pH 7.4, 10 liters) composed of 0.025% of glucose, 0.3% of beef heart infusion, 2% of polypeptone, 0.2% of sodium chloride, 0.2% of dried yeast and 0.4% of disodium hydrogen phosphate was inoculated with *Streptococcus pyogenes* SS 379, type A—40, and the medium was subjected to stationary culture at 37°C for 16 hours. The resulting culture broth was centrifuged to remove the cells. To the filtrate was added solid ammonium sulfate to give 60% saturation, and the mixture was centrifuged at 6,000 × g for 20 minutes. The resulting precipitate was collected and dissolved in a small amount of 0.02 M phosphate buffer (pH 7.0), and the mixture was dialyzed against the same buffer solution for 8 hours and lyophilized to give a powder (1.2 g) containing type A—I esterase.

In the same manner as described above, *Streptococcus pyogenes* 69882, type A—49 and *Streptococcus* sp H36B, type B—I—b were cultivated, and there was obtained each powder containing type A—II or type B esterase, respectively.

## Example 2

Preparation of bacterial cell walls containing "protein-A" (immuno-adsorbent):

A 100 liter fermentor containing a culture medium (pH 7.0, 50 liters) composed of 2% of Bactocasamino acid (manufactured by Difco), 2% of yeast extract, 0.7% of sodium lactate, 2% of sodium glycerophosphate, 0.02% of magnesium sulfate, 0.001% of manganese sulfate, 0.32% of ferric sulfate, 0.32% of sodium citrate, 0.4% of potassium dihydrogen phosphate and 6.25% of disodium hydrogen phosphate was inoculated with *Staphylococcus aureus* Cowan I, and the medium was cultivated for 20 hours at 37°C (aeration rate, 50 liters/min.; agitation speed, 150 r.p.m.). The resulting culture broth was centrifuged to isolate the cells. The cells thus obtained were suspended in water and were disrupted, and the resulting mixture was washed with 1 M aqueous sodium chloride solution (twice) and with water using a centrifuge, and lyophilized to give cell walls containing "protein-A" (58 g).

## Example 3

Preparation of insoluble antibody (immuno-adsorbent):

An anti-human immunoglobulin rabbit antiserum (50 ml), which was prepared by immunizing rabbit with human immunoglobulin, was diluted with 0.1 M potassium phosphate buffer (pH 7.0, 150 ml), and thereto was gradually added a saturated aqueous ammonium sulfate solution (200 ml). The mixture was stirred under ice-cooling for 20 minutes and then centrifuged at 11,000 × g for 20 minutes. The resulting precipitate was collected and dissolved in 0.1 M potassium phosphate buffer (pH 7.0) to make a total volume of 100 ml. To the solution was gradually added a saturated aqueous ammonium sulfate solution (43 ml), and the mixture was stirred under ice-cooling for 20 minutes and again centrifuged. The supernatant fluid was separated and thereto was gradually added a saturated aqueous ammonium sulfate solution (39 ml), and the mixture was stirred under ice-cooling for 20 minutes and then centrifuged. The resulting precipitate was collected and dissolved in 0.02 M potassium phosphate buffer (pH 7.0, 100 ml). The mixture was dialyzed against 0.02 M phosphate buffer (pH 7.0, 10 liters) in a dialysis tube overnight. The dialysis was repeated twice.

The dialyzed inner solution was adjusted with the same buffer solution to give a protein concentration of 1 g/50 ml. The resulting mixture was mixed with an aqueous suspension (20 ml) containing cell walls of *Lactobacillus plantarum* ATCC—8014 (1 g, an insoluble carrier) and 1M sodium acetate buffer (pH 5.0, 10 ml). To the mixture was gradually added with stirring 0.8% aqueous glutaraldehyde solution (120 ml) and the mixture was stirred for additional 2 hours. The reaction mixture was centrifuged at 6,000 × g for 10 minutes. The precipitate was collected and suspended in 0.1% bovine serum albumin (BSA) — 0.9% NaCl — 0.04 M potassium phosphate buffer (pH 7.0, 500 ml) and subjected to the sonication at 19 KC for 30 seconds, and the resulting mixture was centrifuged. The resulting precipitate was collected and added to the same buffer as used above (100 ml), and the mixture was again sonicated and centrifuged. The procedure was repeated twice, and the resulting precipitate was collected and added to the same BSA—NaCl-potassium phosphate buffer as above (100 ml), and the mixture was sonicated again to give a suspension containing bacterial cell wall-bound immunoglobulin rabbit antibody (an insoluble antibody).

The suspension of an insoluble antibody thus obtained was used for the determination of anti-esterase antibody in the following Examples.

4

Example 4

Determination of anti-type A—I esterase antibody:

reagent 1: A solution (10 ml) which was prepared by dissolving the type A—I esterase (50 mg) obtained in Example 1 in water

reagent 2: A suspension (10 ml) which was prepared by suspending the cell walls of *Staphylococcus aureus* Cowan I (750 mg) obtained in Example 2 in water

reagent 3—1: 0.3 mM—DTNB—0.05 M Tris—HCl buffer (pH 8.0)

reagent 3—2: Substrate solution, 10 mM S-acetylthiophenol in ethanol

reagent 3—3: Acetone

buffer solution I: 0.02 M Phosphate buffer (pH 7.0)

buffer solution II: 0.02 M Tris—HCl buffer (pH 8.0)

Sera containing the anti-esterase antibody (d) obtained from patients infected by hemolytic streptococci (two boys of 6 and 4 years old) were used as the test material. The sera were heated at 57°C for 30 minutes.

Each serum was diluted to 20 fold with buffer solution I, and the diluted serum (0.1 ml) was added to a test tube, and thereto was added Reagent 1 (antigen) (50 $\mu$l), and the mixture was incubated at 37°C for 10 minutes to proceed antigen-antibody reaction. To the reaction mixture was added reagent 2 (immuno-adsorbent) (0.1 ml), and the mixture was again incubated at 37°C for 30 minutes. The reaction mixture was washed with buffer solution II twice and centrifuged. The resulting precipitate was collected and suspended in reagent 3—1 (coloring agent) (1 ml) and thereto was added Reagent 3—2 (substrate) (40 $\mu$l), and the mixture was incubated at 37°C for 30 minutes. After adding reagent 3—3 (enzymatic stopping reagent) (2 ml), the reaction mixture was centrifuged at 1,700 × g for 10 minutes. The absorbance of the resulting yellow supernatant fluid was measured at 412 nm in a cell (light path: 1 cm). As a result, the serum from the boy of 6 or 4 years old showed a value of anti-type A—I esterase antibody of 380 or 320, respectively.

The absorbance multiplied by a factor of 1000 was used as the value of antibody (1000 × $A_{412}$/5 $\mu$l serum/30 min. at 37°C).

Example 5

In the same manner as described in Example 4 except that the type A—II esterase obtained in Example 1 was used as reagent 1 and the insoluble antibody obtained in Example 3 was used as reagent 3, two sera obtained from patients infected by hemolytic streptococci (girl of 7 years old and boy of 4 years old) were assayed. As a result, the sera showed a value of anti-type A—II esterase antibody of 852 and 662, respectively.

Example 6

In the same manner as described in Example 4 except that the type B esterase obtained in Example 1 was used as reagent 1, a serum obtained from a patient infected with hemolytic streptococci (girl of 8 years old) was assayed. As a result, the serum showed a value of anti-type B esterase antibody of 924.

Example 7

Differential determination of various types of antibody:

In the same manner as described in Example 4 except that either type A—I, type A—II or type B esterase was added as reagent 1, a serum obtained from a patient infected by hemolytic streptococci (girl of 7 years old) and a serum obtained from a non-infected healthy boy (6 years old) were assayed, by which each of three anti-esterase antibodies was differentially measured. The results are shown in Table 1.

TABLE 1

| | Antibody value | | |
|---|---|---|---|
| Test material | Type A—I | Type A—II | Type B |
| Serum of girl infected by hemolytic streptococci | 970 | 190 | 1250 |
| Serum of non-infected boy | 71 | 55 | 32 |

Example 8

Determination of anti-type A—I esterase antibody:

Sera obtained from patients infected by hemolytic streptococci (girl of 5 years old and boy of 6 years old) were used as the test material. The sera were heated at 57°C for 30 minutes.

Each serum was diluted to 20 fold with buffer solution I as used in Example 4, and the diluted

5

serum (0.1 ml) was added to a test tube, and thereto was added reagent 2 (immuno-adsorbent) (0.1 ml) as used in Example 4, and the mixture was incubated at 37°C for 30 minutes. The resulting precipitate was collected by centrifugation and washed with buffer solution II as used in Example 4 with a centrifuge. To the resulting precipitate was added Buffer solution I (0.1 ml) as used in Example 4, and the mixture was stirred and thereto was further added reagent 1 (50 $\mu$l) as used in Example 4, and the mixture was incubated at 37°C. After 10 minutes, the resulting precipitate was washed with buffer solution II as used in Example 4 with a centrifuge. The activity of esterase in the precipitate was measured in the same manner as in Example 4. As a result, the serum of the girl of 5 years old or the boy of 6 showed a value of anti-type A—I esterase antibody of 720 or 430, respectively.

Example 9

Determination of anti-type A—I esterase antibody:

A serum obtained from a boy (5 years old) infected by hemolytic streptococci was heated at 57°C for 30 minutes. The serum was diluted to 20 fold with buffer solution I as used in Example 4. The diluted serum (0.1 ml) was added to a test tube, and thereto was added reagent 1 (50 $\mu$l) as used in Example 4 and reagent 2 (0.1 ml) as used in Example 4, and the mixture was incubated at 37°C for 10 minutes. The resulting precipitate was treated in the same manner as described in Example 4, and the antibody value was measured likewise. As a result the serum showed an antibody value of 475.

Example 10

Comparison of the methods in Examples 4, 8 and 9:

With respect to several sera obtained from several children patients infected by hemolytic streptococci as mentioned in Table 2, the anti-type A—I esterase antibody was measured in the same manner as described in Examples 4, 8 and 9, respectively. The results are shown in Table 2.

TABLE 2

| Test material | Method for measurement | | |
|---|---|---|---|
| | Example 4 | Example 8 | Example 9 |
| Serum of girl of 4 years old | 100.0 | 89.9 | 92.0 |
| Serum of girl of 5 years old | 100.0 | 92.3 | 90.9 |
| Serum of boy of 5 years old | 100.0 | 87.5 | 93.7 |
| Serum of boy of 4 years old | 100.0 | 98.2 | 88.5 |
| Serum of girl of 6 years old | 100.0 | 90.5 | 95.2 |

[Remark]: The value in the above table shows the relative antibody value when the value in Example 4 is shown as 100.

Example 11

Determination of the anti-type A—I esterase antibody:

The serum obtained from a girl (8 years old) as used in Example 7 was diluted 2 fold successively with buffer solution I as used in Example 4, and values of the anti-type A—I esterase antibody were determined in the same manner as described in Example 4. As shown in Table 3, there was a linear relationship between the antibody values and dilution folds.

**0 061 546**

TABLE 3

| Degree of dilution | 1/16 | 1/8 | 1/4 | 1/2 | 1/1 |
|---|---|---|---|---|---|
| Antibody value | 58 | 121 | 245 | 487 | 975 |

Example 12

Correlation between the value of anti-type A—I esterase antibody and ASO value:

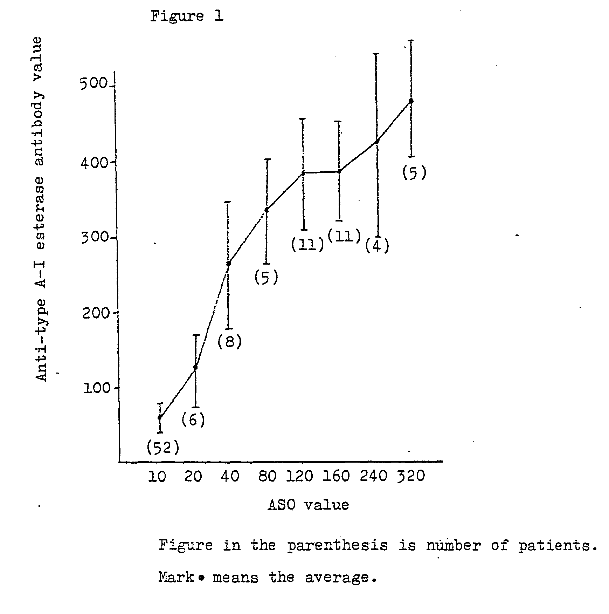

With respect to sera obtained from 102 children patients infected by hemolytic streptococci, the value of anti-type A—I esterase antibody was determined in the same manner as described in Example 4 and the data were compared with the value of an antibody against Streptolysin O (ASO value) determined by the method of Rantz-Randall [cf. Proc. Soc. Exp. Biol. Med., *59,* 22 (1945)]. The correlation of both data is shown in the accompanying Figure 1.

**Claims**

1. A reagent for the determination of an antibody against an esterase from pathogenic streptococci, which comprises a kit of reagents comprising

reagent 1: an esterase (a) from pathogenic streptococci,

reagent 2: a protein (b) which is capable of binding to an antibody (d) against the esterase (a) and is bound to an insoluble carrier, and

reagent 3: a reagent (c) for measuring an activity of the esterase (a).

2. A reagent according to claim 1, wherein the esterase (a) is a member selected from the group consisting of type A—I esterase, type A—II esterase, type B esterase, and a mixture thereof.

3. A reagent according to claim 1, wherein the protein (b) is a member selected from the group consisting of a protein-A and an antibody against the anti-esterase antibody.

4. A reagent according to claim 1, wherein the insoluble carrier is a member selected from the group consisting of bacterial cell walls, glass beads and sephadexes.

5. A reagent according to claim 1, wherein the protein (b) bound to the insoluble carrier is bacterial cell walls containing a protein-A.

6. A reagent according to claim 1, wherein the reagent (c) is a member selected from the group consisting of a combination of S-acetylthiophenol and 5,5'-dithio-bis(2-nitrobenzoic acid), a combination of naphthyl acetate and a diazo-compound, and nitrophenyl acetate.

7. A method for the determination of an antibody against an esterase from pathogenic streptococci in a serum of a patient infected by pathogenic streptococci, which comprises adding reagent 1: an esterase (a) from pathogenic streptococci to a test serum of the patient, and adding thereto reagent 2: a protein (b) which is capable of binding to an antibody (d) against the esterase (a) and is bound to an insoluble carrier, and measuring the activity of esterase contained in the resulting precipitate.

8. A method for the determination of an antibody against an esterase from pathogenic streptococci in a serum of patient infected by pathogenic streptococci, which comprises adding reagent 2: a protein (b) which is capable of binding to an antibody (d) against an esterase (a) from pathogenic streptococci and is bound to an insoluble carrier to a test serum of the patient, adding thereto reagent 1: the esterase (a), and measuring the activity of esterase in the resulting precipitate. esterase in the resulting precipitate.

9. A method for the determination of an antibody against an esterase from pathogenic streptococci in a serum of patient infected by pathogenic streptococci, which comprises adding simultaneously reagent 1: an esterase (a) from pathogenic streptococci and reagent 2: a protein (b) which is capable of binding to an antibody (d) against the esterase (a) and is bound to an insoluble carrier to a test serum of the patient, and measuring the activity of esterase in the resulting precipitate.

**Revendications**

1. Un réactif pour la détermination d'un anticorps contre une estérase de streptocoques pathogènes, qui consiste en un nécessaire de réactifs comprenant:

réactif 1: une estérase (a) de streptocoques pathogènes,

réactif 2: une protéine (b) qui est capable de se fixer sur un anticorps (d) contre l'estérase (a) et qui est fixée sur un support insoluble et

réactif 3: un réactif (c) pour mesurer l'activité de l'estérase (a).

2. Un réactif selon la revendication 1, dans lequel l'estérase (a) est choisi parmi l'estérase type A—I, l'estérase type A—II, l'estérase type B et leurs mélanges.

3. Un réactif selon la revendication 1, dans lequel la protéine (b) est choisie parmi une protéine A et un anticorps contre l'anti-estérase.

7

4. Un réactif selon la revendication 1, dans lequel le support insoluble est choisi parmi les parois de cellules bactériennes, les billes de verre et les Séphadex.

5. Un réactif selon la revendication 1, dans lequel la protéine (b) fixée au support insoluble consiste en parois de cellules bactériennes contenant une protéine A.

6. Un réactif selon la revendication 1, dans lequel le réactif (c) est choisi parmi une combinaison de S-acétylthiophénol et d'acide 5,5'-dithio-bis(2-nitrobenzoïque), une combinaison d'acétate de naphtyle et d'un composé diazo et l'acétate de nitrophényle.

7. Un procédé pour la détermination d'un anticorps contre une estérase provenant de streptocoques pathogènes dans le sérum d'un patient infecté par des streptocoques pathogènes, qui consiste à ajouter le réactif 1: une estérase (a) provenant de streptocoques pathogènes à un sérum d'essai du patient et à y ajouter le réactif 2: une protéine (b) qui est capable de se fixer sur un anticorps (d) contre l'estérase (a) et qui est fixée sur un support insoluble, et à mesurer l'activité d'estérase contenu dans le précipité résultant.

8. Un procédé pour la détermination d'un anticorps contre une estérase provenant de streptocoques pathogènes dans le sérum d'un patient infecté par des streptocoques pathogènes qui consiste à ajouter le réactif 2: une protéine (b) qui est capable de se fixer sur un anticorps (d) contre une estérase (a) provenant de streptocoques pathogènes et qui est fixée sur un support insoluble à un sérum d'essai du patient, à y ajouter le réactif 1: l'estérase (a), et à mesurer l'activité d'estérase dans le précipité résultant.

9. Un procédé pour la détermination d'un anticorps contre une estérase provenant de streptocoques pathogènes dans le sérum d'un patient infecté par des streptocoques pathogènes, qui consiste à ajouter simultanément le réactif 1: une estérase (a) provenant de streptocoques pathogènes et le réactif 2: une protéine (b) qui est capable de se fixer sur un anticorps (d) contre l'estérase (a) et qui est fixée sur un support insoluble à un sérum d'essai du patient et à mesurer l'activité d'estérase dans le précipité résultant.

**Patentansprüche**

1. Reagenz zur Bestimmung eines Antikörpers gegen eine Esterase aus pathogen Streptococcen, bestehend aus einem Reagentiensatz, enthaltend
Reagenz 1: eine Esterase (a) aus pathogen Streptococcen,
Reagenz 2: ein an einen unlöslichen Träger gebundenes Protein (b), das sich an einen gegen die Esterase (a) gerichteten Antikörper (d) binden kann, und
Reagenz 3: ein Reagenz (c) zur Bestimmung einer Aktivität der Esterase (a).

2. Reagenz nach Anspruch 1, wobei die Esterase (a) eine Esterase vom Typ A—I, Typ A—II, Typ B oder deren Gemisch ist.

3. Reagenz nach Anspruch 1, wobei das Protein (b) ein Protein-A oder ein Antikörper gegen den Anti-Esterase-Antikörper ist.

4. Reagenz nach Anspruch 1, wobei der unlösliche Träger aus der Gruppe der Bakterienzellwände, Glaskügelchen und Sephadexe ausgewählte ist.

5. Reagenz nach Anspruch 1, wobei das an den unlöslichen Träger gebundene Protein (b) bakterienzellwände sind, die ein Protein-A enthalten.

6. Reagenz nach Anspruch 1, wobei das Reagenz (c) eine Kombination von S-Acetylthiophenol und 5,5'-Dithiobis-(2-nitrobenzoesäure), eine Kombination von Naphtylacetat und einer Diazoverbindung, oder Nitrophenylacetat ist.

7. Verfahren zur Bestimmung eines Antikörpers gegen eine Esterase aus pathogenen Streptococcen im Serum eines durch pathogene Streptococcen infizierten Patienten, dadurch gekennzeichnet, daß man ein Testserum des Patienten mit einem Reagenz 1, nämlich einer Esterase (a) aus pathogenen Streptococcen und sodann einem Reagenz 2, nämlich einem an einen unlöslichen Träger gebundenen Protein (b), versetzt, das in der Lage ist, sich an einen gegen die Esterase (a) gerichteten Antikörper (d) zu binden, und die in der gebildeten Fällung enthaltene Esteraseaktivität bestimmt.

8. Verfahren zur Bestimmung eines Antikörpers gegen eine Esterase aus pathogen Streptococcen im Serum eines durch pathogene Streptococcen infizierten Patienten, dadurch gekennzeichnet, daß man ein Testserum des Patienten mit einem Reagenz 2 versetzt, nämlich einem an einen unlöslichen Träger gebundenen Protein (b), das in der Lage ist, sich an einen gegen eine Esterase (a) aus pathogen Streptococcen gerichteten Antikörper (d) zu binden, sodann das Reagenz 1 zugibt, nämlich die Esterase (a), und die Aktivität der Esterase in der erhaltenen Fällung bestimmt.

9. Verfahren zur Bestimmung eines Antikörpers gegen eine Esterase aus pathogenen Streptococcen im Serum eines durch pathogene Streptococcen infizierten Patienten, dadurch gekennzeichnet, daß man ein Testserum des Patienten gleichzeitig mit einem Reagenz 1, nämlich einer Esterase (a) aus pathogenen Streptococcen, und einem Reagenz 2, nämlich einem an einen unlöslichen Träger gebundenen Protein (b), das in der Lage ist, sich an einen gegen die Esterase (a) gerichteten Antikörper (d) zu binden, versetzt und die Aktivität der Esterase in der erhaltenen Fällung bestimmt.

Figure 1

Figure in the parenthesis is number of patients.

Mark • means the average.